# EUROPEAN PATENT SPECIFICATION

(11) **EP 1 226 228 B1**
(45) Date of publication and mention of the grant of the patent: **14.04.2010**
(21) Application number: 00973025.0
(22) Date of filing: 02.11.2000
(51) Int. Cl.: C12M 1/34, C12M 3/04

(54) **A CELL MIGRATION AND CHEMOTAXIS CHAMBER**
ZELLMIGRATIONS- UND CHEMOTAXISKAMMER
DISPOSITIF DE MIGRATION CELLULAIRE ET DE CHIMIOTAXIE

(30) Priority: 03.11.1999 GB 9925904
(43) Date of publication of application: 31.07.2002
(73) Proprietor: The Queens University of Belfast, Belfast BT7 1NN (GB)
(72) Inventor: NELSON, John, Belfast BT9 7BU (GB); GAMBLE, Harold, Drumore, Co Down BT25 1HA (GB); CAMPBELL, Denis, Lisburn, Co Antrim BT27 4QX (GB)
(74) Representative: Main, Malcolm Charles
(86) International application number: PCT/GB2000/004213
(87) International publication number: WO 2001/032827

(56) References cited:
- US-A- 4 729 949
- US-A- 4 895 805

## Description

This invention relates to a cell migration and chemotaxis chamber for use in investigating and measuring the migratory and invasive behaviour of cells, and in the investigation of potential drugs, and to a method for producing such a chamber.

It is recognised that many of the most successful therapeutic drugs have, in the past, been found serendipitously. Given the millions of chemicals synthesised or extracted over the years it is expected that unsuspected potential drugs are to be found in this vast resource. To address this problem, many labs began to adopt a stochastic screening programme in which libraries of chemical compounds or natural products are tested for activity in robotic laboratories.

For example, the National Cancer Institute holds 130,000 discrete extracts from plants, marine invertebrates and micro-organisms which it tests for effects on the growth of cancer cells or HIV (in its anti-AIDS programme). However, apart from the important high through put screening methods for cell growth and cell death, the only other such screening methods available are dedicated to assaying drug effects on single oncogenes - for example the ability of a drug to bind to a cell surface receptor like the EGF receptor.

There is currently no automated screening method for cell migration nor for the invasive behaviour of cancer cells.

Assessment of the effects of potential drugs on cell migration is important in diseases where angiogenesis is involved. A second aspect of cell migratory behaviour, invasiveness, is important in cancer metastasis, where cancer cells migrate into and invade new tissue. Assessment of migratory behaviour is also important in basic research areas such as developmental biology, immunology and the like.

Angiogenesis is the formation of new blood vessels. Angiogenesis is utterly dependent upon the directional migration of endothelial cells towards the source of some angiogenic stimulating factor (chemotaxis). It is widely accepted that angiogenesis is an important target in a number of common diseases. For example, cancer metastasis, diabetic retinopathy, psoriasis and rheumatoid arthritis are all dependent on angiogenesis for their progression and could be treated by agents which inhibit angiogenesis. On the other hand, it would be desirable to screen for factors that promote angiogenesis in the treatment of coronary artery and peripheral artery disease.

In vivo tests for angiogenic factors are expensive, time-consuming and provide few replicates (for example, the rabbit corneal pouch assay and the chick chorioallanoic membrane assay). Current in vitro screens for potential anti-angiogens are mostly dependent on measuring inhibition of cell migration towards an angiogenic stimulus such as cancer cell growth factors like EGF. However, these assays do not lend themselves to automation. Furthermore, even for manual basic research purposes the current assays are inefficient. Three main assays are in use:
1. The Boyden Chamber (and its modifications) consists of an upper chamber separated from a bottom chamber by a plastic membrane (typically polycarbonate) which has 8 µm pores in it. To assess chemotaxis, tissue culture medium containing chemoattractant is placed in the bottom chamber and medium containing cells is placed in the top chamber. The cells will then migrate towards the source of chemoattractant by squeezing through the pores (an animal cell is 20µm in diameter: cells cannot simply fall through the pores, but must use energy to locomote through). To measure chemoinvasion, the pores are first plugged with a extracellular matrix protein (like collagen). In this case, the cells must degrade the protein before they can pass through the pores.

### 1.2 Advantages

A large number of cells are monitored in each Boyden Chamber (1000-1 million), giving a more reliable estimate of drug effects. The assay does measure directional migration ("Chemotaxis"). Many replicates are possible, particularly when using inexpensive plastic disposable versions of the Boyden Chamber.

### 1.3 Problems

1.3a The main problem with the Boyden Chamber method is its quantification. Cells which migrate do not drop to the bottom of the chamber, but instead stick to and colonise the underside of the plastic membrane. In order to quantify migrated cells, the cells on the upper surface must first be carefully removed by scraping with a cotton bud. The membranes are delicate and easily punctured. The membrane is then stained with crystal violet (or some other cell stain) and the cells are either counted manually (using a microscope) or the cell-associated stain is re-dissolved and measured in a spectrophotometer. Manual cell counting is tedious and prone to operator error. Colorimetric assay of dye depends on there being sufficient numbers of invading cells (at least 1000) and is subject to interference because such dyes often bind to the proteins used to plug the pores, leading to unacceptably high background.

Cells will eventually fully colonise the underside of the membrane, and it is at this point that they start to drop off and fall to the bottom chamber where they can be stained and counted more easily. However, this typically takes a week, by which time a great deal of cell division has occurred, particularly in the bottom chamber containing the growth factor. So, in this case, the number of cells in the bottom is a result of not just migration but also cell division.

1.3b) A second problem with the Boyden Chamber is that it is a 'snap-shot'. That is, one knows how many cells migrate under treatment conditions compared withy a control but one has no idea of how fast the cells migrate. For example in a 48 hour migration assay both control and treatment membranes are fixed at 48 hours. All the cells might have migrated in the first 2 hours with no further movement in the following 46 hours, but the Boyden Chamber Assay will give no information on this.

1.3c) The polycarbonate membrane fabrication process does not guarantee a fixed number of pores per chamber.

1.3d) Re-usable Boyden Chambers are difficult to reassemble - air bubbles often become trapped and it is easy to puncture new membranes. Disposable Boyden Chambers are similarly prone to membrane punctures.

### 2) The Dunn Chamber

The Dunn Chamber aims to address the problem of cell migration speeds. It consists of a specially constructed microspore slide with a central circular sink and a concentric annular moat. In this assay cells migrate on a coverslip (which is placed inverted on the Dunn Chamber) towards a chemotactic chemical. The cells are monitored over-night using a phase-contrast microscope fitted with a video camera connected to a computer with image-grabber board. The microscope must have a heated stage and is usually fitted with an automatic electronic iris on its condenser.

### 2.1 Advantages

The Dunn Chamber measures directional migration continuously by means of time-lapse.

### 2.2) Problems

2.2a) A major problem with the Dunn Chamber Assay is that a very small number of cells are monitored (typically ten). This is a very small sample and average behaviour of this small sample may not therefore be typical of the population as a whole.

2.2b) A second major problem is that replication is very restricted. Each control chamber and each treatment chamber must be viewed in separate microscopes, each one similarly equipped with a camera and computer. Each videomicroscopy unit would cost £20.000. So, to do three controls and three treatments would require an investment of £120,000, as experiments should ideally be performed on the same cell harvest and on the same day.

2.2c) Interpretation of the stored images requires bespoke software and a skilled operator. The present version of software requires manual logging of each cell's centre on each frame of the time-lapse video. This is highly time-consuming.

### 3. The Albrecht-Buhler Phagokinetic Track Assay .

An earlier cell motility/migration assay is, like the Dunn Chamber, dependent on measuring the tracks of cells. In this assay, coverslips are coated with colloidal gold particles. Cells are allowed to locomote over this gold lawn overnight. The cells clear tracks during their migration and the area of tracks gives an indication of the speed of motility.

### 3.1) Advantages

Many replicates are possible due to the cheapness of the assay. Only one microscope is required. Analysis is time-consuming unless automated image analysis is used. Although not monitored continuously, the cells leave a record of their overnight activity.

### 3.2) Problems

3.2a) The main problem with the Phagokintetic Track Assay is that it does not measure chemotaxis. Movement may be stimulated or inhibited, but the movement is random.

3.2b) A second major problem is that the cells ingest some of the gold particles. This is clearly not physiologically appropriate and it is unknown what effect this has on cells' behaviour or responses to added agents.

3.2c) A third difficulty with the assay is that the physical chemistry required to produce the gold colloid is difficult to replicate and scale-up. Differing densities of gold coating and variation in particle size are encountered from batch to batch.

3.3d) Finally, cells often back-track, covering the same path more than once. So the observed track is not necessarily a true record of the cell's activity.

US 4,729,949 discloses a device to separate cells according to their size. The device of US 4, 729, 349 comprises a cell carrier which has an array of cell receiving holes, the location of which is fixed and known, the holes having preselected configurations such that cells of interest become supported in the holes, one cell per hole at a fixed address.

US 4,895,805 relates to a cell manipulation apparatus comprising a carrier plate for moving cells of interest into specific chambers of a chamber plate in which cell fusion can be effected. Communicating holes and slits are provided in the cavities of the carrier plate and chambers of the chamber plate to enable liquid pressure to be used to transfer cells from the cavities of the carrier plate to the chambers of the chamber plate.

An object of the present invention is to solve or mitigate some of the above problems, particularly those relating to low numbers of replicates, expense and requirement for labour intensive and skilled interpretation and technical expertise.

According to the present invention there is provided a cell migration assessment device as claimed in claim 1.

Preferably, an array of passageways is provided, suitably a 10 x 10 array.

The planar member is preferably a silicon wafer, which may suitably be about 525 microns thick.

Said passageways are preferably etched to have the form of a hopper extending from a top surface and terminating in an aperture. Typically, the top of the hopper will be a square of 700 microns per side, and the aperture will be a pore of about 8 microns per side.

Preferably, the device comprises the silicon wafer held between an upper cup into which a cell culture may be introduced, and a lower stand suitable for being received in a cluster plate well.

The silicon wafer is provided with means for sensing the passage of cells through the apertures. Said means may detect change in a physical parameter, such as an electrical or optical criterion, as the cells pass. Arrays of conductors are formed on the upper and lower surfaces of the wafers and disposed for sensing changes in an electrical parameter (such as resistance or capacitance) between conductors as cells pass through the apertures.

Described is a method of making a planar member for use in a cell migration assessment device, the method comprising the steps of providing a planar member in the form of a wafer;
applying a resist pattern to an upper surface of the wafer to define an array of relatively large surface areas;
etching the upper surface for a time sufficient to expose the silicon in said array;
applying a resist pattern to a lower surface of the wafer to define a matching array of relatively small surface areas;
etching the lower surface for a time sufficient to expose the silicon in said surface areas; and
etching the exposed silicon at both surfaces to produce hopper-shaped openings from the top surface and connecting holes or pores through the bottom surface.

Typically, the wafer is of silicon, and the method is carried out using techniques known from processing of silicon integrated circuits.

The method may further comprise laying down a pattern of conductors on one or both surfaces for use in measuring an electrical parameter associated with each hole.

The invention further provides the use of a device of the invention for the selection of highly motile or invasive cells from a mixed population. This use can typically encompass clonal selection of cells.

Embodiments of the present invention will now be described, by way of example only, with reference to the drawings, in which:
Figure 1 is a perspective view of a chamber in accordance with the present invention;
Figure 2 illustrates the chamber of Figure 1 in use;
Figure 3 is a schematic cross section corresponding to Figure 2;
Figures 4a to 4e represents schematically stages in the production of part of the apparatus in Figures 1-3 ; and
Figure 5 is a schematic plan view.

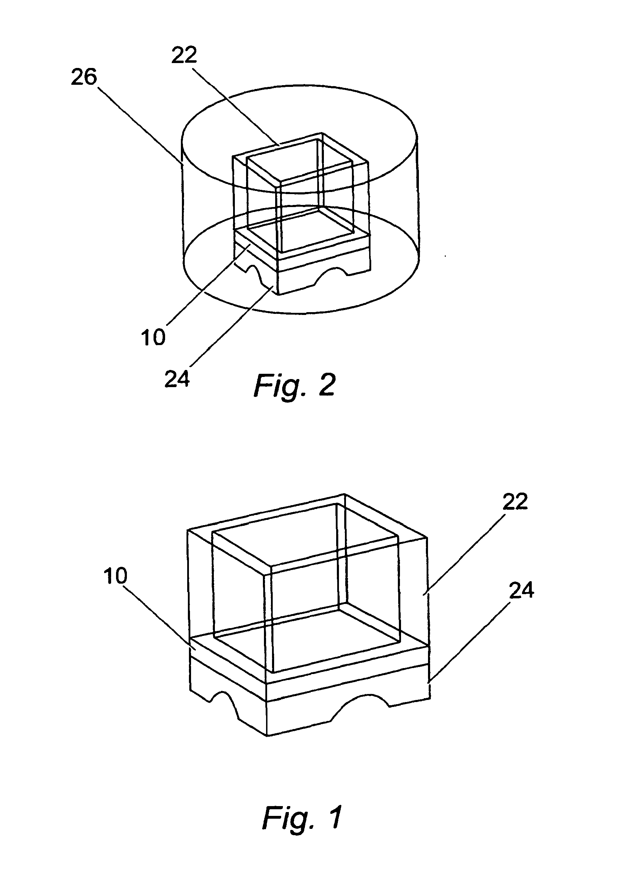

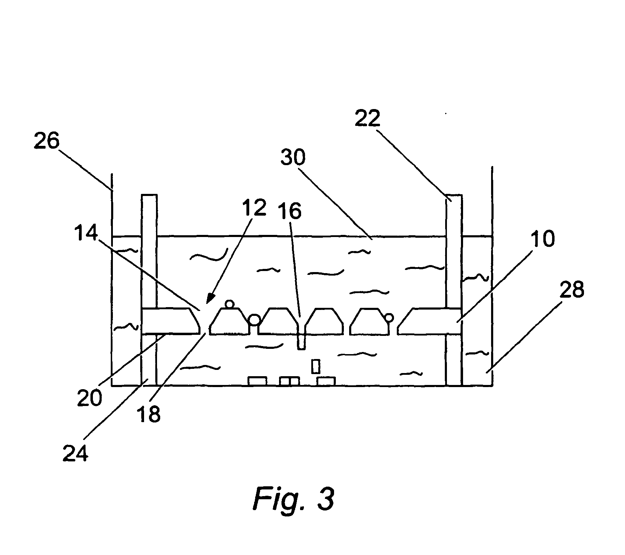

Referring to Figures 1-3, a cell migration and chemotaxis chamber consists of a silicon wafer 10 in which a 10mm X 10mm array of one hundred holes 12 have been etched. On the upper surface 14 the holes are 700µm square cross-section leading, via a hollow inverted pyramidal 'hopper' 16, to a 8µm diameter exit pore 18 on the lower surface 20. The wafer 10 is sealed into a glass or plastic chamber, with an upper cup 22 and a lower stand 24. The chamber is designed to fit into standard disposable tissue culture cluster plates (12-well), and is used like a Boyden Chamber. The chamber is placed in a cluster plate well 26 containing the medium plus chemoattractant 28 and the cells in control or drug-containing medium 30 is placed in the upper cup 22 above the silicon wafer 10. At the end of the experiment, the chamber is simply lifted out of the plate and the migrated cells (left behind in the cluster plate well 26) are fixed, stained and cell-associated stain can be re-dissolved and read in a spectrophotometer.
The foregoing embodiment has the following advantages:

The cells do not stick to the lower surface 20 of the silicon wafer 10, but instead drop to the floor of the cluster plate well 26. This means that the upper surface 14 does not need to be scraped with a cotton bud (as is the case with the Boyden Chamber).

Since all the migrated cells are at the bottom of the cluster plate well 26, it is easy to fix and stain them allowing colorimetric assay of cell numbers.

Since the cells do not stick to the undersurface 20 of the wafer 10, non-specific staining of protein coatings does not interfere (as is the case with the Boyden Chamber).

Cells of interest will typically adhere to a plane silicon surface. It is believed that they do not adhere to the underside of the wafer 10 because of the hole geometry. The mechanism is not at present fully understood, but it is thought that the exit pore 18 joining the lower face 20 in a relatively sharp edge is of significance.

A glass chamber of this embodiment can be re-used without difficult re-assembly (as is the case with the Boyden Chamber). To remove cells it is boiled in 5% tissue culture detergent then washed in distilled water. It can then be re-sterilised by autoclave. If production were scaled up and wafers were encased in plastic chambers, they could be made cheap enough to be disposable.

Each wafer 10 has exactly the same number of holes 12 with very precise dimensions, making chemoattractant gradients between upper and lower wells more predictable and uniform (than is the case with the Boyden Chamber).

The uniformity of numbers, and consistent geometry of the hoppers 16 and pores 18, would allow more consistent and reproducible protein coating or plugging of the wafer and/or pores 18.

The consistent geometry of the array would allow for robotic dispensing of protein solutions into each hopper using a suitable array of micro-pipettes.

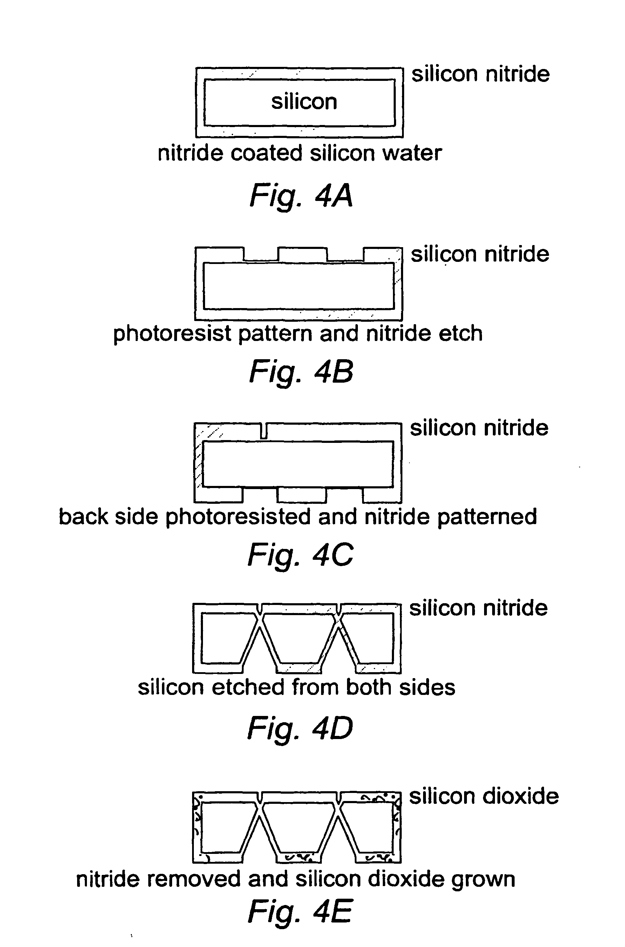

Referring now to Figure 4 one example of a process for producing the silicon wafer of the foregoing embodiment will now be described.

The starting material (Fig. 4A) was 4 inch diameter, n-type silicon wafers, <100> orientation, 9-16 ohm-cm resistivity, polished both sides and 525 µm thick.

The wafers were cleaned and coated with a 220 nm thick layer of LPCVD silicon nitride on all sides. Positive photoresist was deposited on the top side, prebaked and exposed to UV light through an appropriately patterned chrome photo mask. The mask is aligned so that the edges of the square patterns lie along the 100 directions. The exposed photoresist was developed to remove the resist from the square patterns. After baking the photoresist, the square patterns were reactive ion etched through the exposed silicon nitride on the top side. The photoresist was removed, and the wafers cleaned in a megasonic bath and then annealed at 1000°C in N2 for ten minutes (Fig. 4B).

The bottom side of the wafer was coated with photoresist, which was pre-baked and exposed to UV light through a second patterned chrome photo mask. This second mask was aligned to the pattern on the other side of the wafer using an Electronic Visions EV420 double side mask aligner. The pattern was reactive ion etched through the nitride to expose the silicon (Fig. 4C).

The silicon was anisotropically etched in a ternary mixture of KOH (50gm), IPA (51 cc) and DI-H20(162 cc) at 80 ± 1°C in a lufran super bowl reflux system. This etch attacks the <100> planes at a much faster rate than the <111> planes to produce inverted square pyramid-like wells. The etching which took place from both sides was stopped once the wells from the two sides had met (Fig. 4D). This took about 5 hours. The silicon nitride layer was removed by wet chemistry and the wafer oxidised at 1050°C for one hour to grow a surface silicon dioxide layer of 100 nm (Fig. 4E). The silicon wafer was then diced into 10mm square die to produce a number of Chambers.
A second type of a chamber in accordance with the invention will now be described.

This is designed to allow remote and continuous monitoring of cell migration with time. Emergence of a cell though each pore might be monitored in several different ways. Optical methods might be possible, but since cells are transparent it is likely that he cells would have to be dyed or fluorescently labelled which would be non-physiological. It is likely that electrical methods would be the most economical way to do this. For example changes in electrical resistance or capacitance would be sensed as a cell blocked the pore.

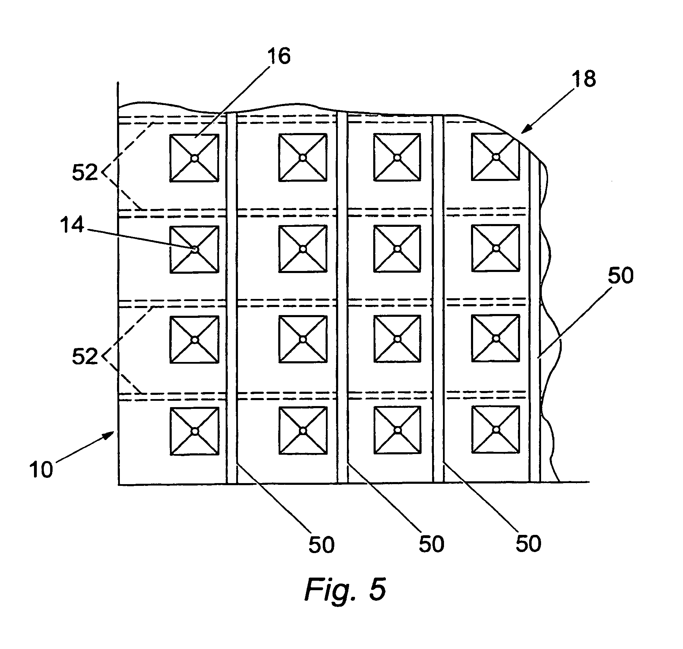

Based on this idea, the chamber further (Fig. 5) consists of a silicon chip. In Figure 5, parts similar to those of Figures 1-3 are denoted by the same reference numerals. Upper and lower sets of conductors 50 and 52 are deposited on the surfaces 14 and 20 to allow detection of cells as they pass through each of the lower 8 µm exit holes 18. Detection may be most easily achieved by measuring the change in electrical resistance as the cell blocks the hole 18 separating adjacent ones of the upper and lower conductors 50, 52. This could be detected as a resistance to the flow of electrical current between top and bottom conductors. The current would not be continuous, but only applied momentarily when the array is being 'interrogated'. The array would be repeatedly scanned so that data is logged, with time, for each individual hole.

This arrangement means that cell migration can be monitored automatically and continuously during the experiment, rather than providing a mere 'snap-shot'

Cells do not have to be stained, removing the need for calibration of each cell-line (various cell types take up varying amounts of dye per cells) and removing interference from staining of coating protein.

The remote sensing capability of this embodiment combined with the low costs of silicon chip manufacturing mean that this method could be used in robotic drug screening.

## Claims

1. A cell migration assessment device comprising; a planar member having, in use, an upper surface and a lower surface, wherein said planar member has at least one passageway extending from the upper surface to an aperture in the lower surface said passageway having a cross-sectional width having a minimum dimension less than the cross-sectional width of cells of interest through which such cells can be caused to locomote, said passageway being formed by etching a material to form the aperture In the lower surface, the aperture having a geometry which, in use, prevents adherence of the cells to the lower surface of the planar member, wherein the planar member is provided with arrays of conductors formed on the upper and lower surface of the member and disposed for sensing changes in an electrical parameter between conductors as cells pass through the aperture(s) such that the emergence of a cell through said passageway(s) can be monitored.

2. The device as claimed In claim 1 wherein the aperture has a geometry wherein the aperture joins the lower surface to form a relatively sharp edge.

3. The device as claimed in claim 1 or claim 2 wherein an array of passageways is provided.

4. The device as claimed in any preceding claim wherein the planar member is a silicon wafer.

5. The device as claimed in any preceding claim wherein said passageway(s) are etched to have the form of a hopper extending from a top surface and terminating in an aperture which forms said minimum dimension.

6. A device as claimed in claim 4 wherein the silicon wafer is held between an upper cup into which cell culture may be introduced, and a lower stand suitable for being received In a cluster plate well.

7. A device according to claim 4 wherein the silicon wafer is 525µm thick.

8. Use of a device as claimed in any one of claims 1 to 7 for selection of highly motile or invasive cells.

9. Use of a device as claimed in claim 8, in which the upper surface of the planar member is contacted with a medium containing said cells, and the lower surface of the planar member is contacted with a medium containing a chemoattractant, and in which the quantity of the cells passing through the planar member in a given time is monitored.

## Patentansprüche

1. Eine Vorrichtung zur Einschätzung von Zellmigration, die Folgendes beinhaltet: ein planares Element, das bei Gebrauch eine obere Oberfläche und eine untere Oberfläche aufweist, wobei das planare Element mindestens einen Durchgang aufweist, der sich von der oberen Oberfläche zu einer Öffnung in der unteren Oberfläche erstreckt, wobei der Durchgang eine Querschnittsbreite aufweist, deren kleinste Abmessung kleiner als die Querschnittsbreite von Zellen von Interesse ist, durch die sich fortzubewegen derartige Zellen veranlasst werden können, wobei der Durchgang durch Ätzen eines Materials zum Bilden der Öffnung in der unteren Oberfläche gebildet ist, wobei die Öffnung eine Geometrie aufweist, die bei Gebrauch ein Anhaften der Zellen an der unteren Oberfläche des planaren Elements verhindert, wobei das planare Element mit Anordnungen von Leitern versehen ist, die auf der oberen und unteren Oberfläche des Elements gebildet und eingerichtet sind, um, wenn Zellen die Öffnung(en) durchlaufen, Änderungen eines elektrischen Parameters zwischen Leitern wahrzunehmen, so dass das Austreten einer Zelle durch den Durchgang/die Durchgänge überwacht werden kann.

2. Vorrichtung gemäß Anspruch 1, wobei die Öffnung eine Geometrie aufweist, wobei die Öffnung an der unteren Oberfläche ansetzt, um eine relativ scharfe Kante zu bilden.

3. Vorrichtung gemäß Anspruch 1 oder Anspruch 2, wobei eine Anordnung von Durchgängen bereitgestellt ist.

4. Vorrichtung gemäß einem der vorhergehenden Ansprüche, wobei das planare Element eine Siliziumscheibe ist.

5. Vorrichtung gemäß einem der vorhergehenden Ansprüche, wobei der Durchgang/die Durchgänge geätzt ist/sind, um die Form eines Trichters aufzuweisen, der sich von einer Oberseitenoberfläche erstreckt und in einer Öffnung endet, die die kleinste Abmessung bildet.

6. Vorrichtung gemäß Anspruch 4, wobei die Siliziumscheibe zwischen einer oberen Schale, in die eine Zellkultur eingeführt werden kann, und einem unteren Fuß, der dazu geeignet ist, in einer Sammelplattenmulde aufgenommen zu werden, gehalten wird.

7. Vorrichtung gemäß Anspruch 4, wobei die Siliziumscheibe 525 µm dick ist.

8. Eine Verwendung einer Vorrichtung gemäß einem der Ansprüche 1 bis 7 für eine Auswahl von hochgradig freibeweglichen oder invasiven Zellen.

9. Verwendung einer Vorrichtung gemäß Anspruch 8, wobei die obere Oberfläche des planaren Elements mit einem Medium, das die Zellen enthält, in Kontakt gebracht wird und die untere Oberfläche des planaren Elements mit einem Medium, das einen chemischen Lockstoff enthält, in Kontakt gebracht wird und wobei die Menge der Zellen, die das planare Element in einer gegebenen Zeit durchlaufen, überwacht wird.

## Revendications

1. Un dispositif d'évaluation de migration cellulaire comprenant :
un élément planaire ayant, à l'utilisation, une surface supérieure et une surface inférieure, où ledit élément planaire a au moins une voie de passage s'étendant de la surface supérieure à une ouverture dans la surface inférieure de ladite voie de passage ayant une largeur en coupe transversale ayant une dimension minimum inférieure à la largeur en coupe transversale de cellules d'intérêt dans laquelle de telles cellules peuvent être amenées à circuler, ladite voie de passage étant formée en gravant un matériau pour former l'ouverture dans la surface inférieure, l'ouverture ayant une géométrie qui, à l'utilisation, empêche l'adhérence des cellules sur la surface inférieure de l'élément planaire, où l'élément planaire est pourvu de réseaux de conducteurs formés sur la surface supérieure et la surface inférieure de l'élément et disposés de façon à détecter des changements dans un paramètre électrique entre des conducteurs lorsque des cellules passent à travers l'ouverture (les ouvertures) de façon à ce que l'émergence d'une cellule dans ladite (lesdites) voie(s) de passage puisse être surveillée.

2. Le dispositif tel que revendiqué dans la revendication 1 où l'ouverture a une géométrie selon laquelle l'ouverture rejoint la surface inférieure pour former une arête relativement vive.

3. Le dispositif tel que revendiqué dans la revendication 1 ou la revendication 2 où un réseau de voies de passage est prévu.

4. Le dispositif tel que revendiqué dans n'importe quelle revendication précédente où l'élément planaire est une plaquette de silicium.

5. Le dispositif tel que revendiqué dans n'importe quelle revendication précédente où ladite (lesdites) voie(s) de passage est (sont) gravée(s) de façon à avoir la forme d'une trémie s'étendant depuis une surface de dessus et se terminant en une ouverture qui forme ladite dimension minimum.

6. Un dispositif tel que revendiqué dans la revendication 4 où la plaquette de silicium est tenue entre une coupelle supérieure à l'intérieur de laquelle une culture de cellules peut être introduite, et un support inférieur qui convient pour être reçu dans un puits de plaque pour amas.

7. Un dispositif selon la revendication 4 où la plaquette de silicium a une épaisseur de 525 µm.

8. Utilisation d'un dispositif tel que revendiqué dans n'importe laquelle des revendications 1 à 7 pour la sélection de cellules hautement motiles ou invasives.

9. Utilisation d'un dispositif tel que revendiqué dans la revendication 8, où la surface supérieure de l'élément planaire est mise en contact avec un milieu contenant lesdites cellules, et la surface inférieure de l'élément planaire est mise en contact avec un milieu contenant un agent chimiotactique, et où la quantité des cellules qui traversent l'élément planaire dans un temps donné est surveillée.
